(19) Europäisches Patentamt
European Patent Office
Office européen des brevets

(11) EP 0 754 667 A2

(12) **EUROPÄISCHE PATENTANMELDUNG**

(43) Veröffentlichungstag:
**22.01.1997 Patentblatt 1997/04**

(51) Int. Cl.[6]: **C07C 43/15**, C07C 69/00

(21) Anmeldenummer: **96111234.9**

(22) Anmeldetag: **12.07.1996**

(84) Benannte Vertragsstaaten:
**BE DE ES FI FR GB IT NL SE**

(30) Priorität: **20.07.1995 DE 19526501**

(71) Anmelder: **BASF AKTIENGESELLSCHAFT**
**67056 Ludwigshafen (DE)**

(72) Erfinder:
- **Oftring, Alfred, Dr.**
**67098 Bad Dürkheim (DE)**
- **Aus dem Kahmen, Martin, Dr.**
**67071 Ludwigshafen (DE)**
- **Ehle, Beate, Dr.**
**67059 Ludwigshafen (DE)**
- **Baur, Richard, Dr.**
**67112 Mutterstadt (DE)**
- **Klingelhöfer, Paul, Dr.**
**68165 Mannheim (DE)**
- **Schneider, Jürgen, Dr.**
**67251 Freinsheim (DE)**
- **Ruland, Alfred, Dr.**
**69198 Schriesheim (DE)**
- **Trapp, Horst, Dr.**
**68723 Plankstadt (DE)**
- **Ott, Christian, Dr.**
**67346 Speyer (DE)**

(54) **Hydroxymischether erhalten durch Ringöffnung von Epoxiden ungesättigter Fettsäureester mit Polyglykolethern und ihre Verwendung als biologisch abbaubaren Entschäumer**

(57) Hydroxymischether, dadurch erhältlich, daß man Epoxide von ungesättigten Fettsäureestern einer ringöffnenden Umsetzung mit Polyglykolethern I

$$R^1\text{—O—}(CH_2\text{—}CHR^2\text{—O})_n\text{—H} \qquad (I)$$

bei denen $R^1$ für Wasserstoff, $C_1$- bis $C_{30}$-Alkyl oder $C_8$- bis $C_{30}$-Alkenyl steht, $R^2$ Wasserstoff, Methyl oder Ethyl bezeichnet und n eine Zahl von 1 bis 50 für den Fall $R^1 \neq$ Wasserstoff oder eine Zahl von 2 bis 100 für den Fall $R^1$ = Wasserstoff bedeutet, in Gegenwart von Lewis-Säuren mit Ausnahme von Protonen, insbesondere in Gegenwart von Bortrifluorid, Bortrifluorid-Addukten oder Fluoroborsäure, unterwirft.

Diese Hydroxymischether eignen sich als Entschäumer insbesondere in der Nahrungsmittelindustrie und bei Fermentationsprozessen, in Wasch- und Reinigungsmitteln, bei der Textilherstellung, bei der Leder- und Pelzherstellung, bei der Papierherstellung, bei der Metallbearbeitung und bei der Erdölgewinnung.

EP 0 754 667 A2

## Beschreibung

Die vorliegende Erfindung betrifft neue durch ringöffnende Umsetzung von Epoxiden von ungesättigten Fettsäureestern mit Polyglykolethern erhältliche Hydroxymischether, ein Verfahren zu deren Herstellung sowie ihre Verwendung als biologisch abbaubarer Entschäumer.

Entschäumer, auch Schauminhibitoren, Antischaummittel oder Schaumdämpfer genannt, sind Verbindungen, die schäumenden Flüssigkeiten in der Technik zugesetzt werden, um deren Schaumbildung zu reduzieren oder zu verhindern. Entschäumer sind entweder grenzflächenaktive Stoffe, die die Schaumbildung an der Grenzfläche verdrängen, ohne Schaum zu erzeugen, oder aber Verbindungen, die die Oberflächenspannung des Wassers erhöhen, z.B. natürliche Fette, Öle oder Fettalkohole.

Der Einsatz von Entschäumern ist sowohl im Haushalt, beispielsweise in maschinellen Geschirrspülmitteln oder in Textilwaschmitteln, als auch in der Industrie, beispielsweise bei der Herstellung von Papier oder Zucker, unumgänglich. In schaumarmen Reinigungsmitteln wird das starke Schaumvermögen der darin enthaltenen anionischen Tenside in der Regel durch die Mitverwendung langkettiger Seifen herabgesetzt. Auch Entschäumer auf Siliconbasis haben an Bedeutung gewonnen. Im Bereich der maschinellen Flaschenreinigung und anderer industrieller Anwendungen werden häufig Polyethylen- oder Polypropylenglykolether als Entschäumer eingesetzt; diese Verbindungen sind zwar einfach und kostengünstig herstellbar, zeichnen sich allerdings durch unzureichende biologische Abbaubarkeit und oft durch unbefriedigende Entschäumerwirkung aus.

Aus der DE-A 40 38 608 (1) ist die Verwendung von Esterpolyolen enthaltenden Reaktionsmischungen als schaumdämpfende Zusatzstoffe bei der Herstellung und Verarbeitung von Nahrungsmitteln und bei Fermentationsprozessen bekannt. Diese Esterpolyole werden durch ringöffnende Umsetzung epoxidierter Carbonsäureester u.a. mit Alkoholen, die auch teilweise verethert sein können, in Gegenwart von sauren Katalysatoren wie starken Mineralsäuren, z.B. Schwefelsäure, hergestellt.

Die WO-A 92/19577 (2) betrifft ein Verfahren zur Herstellung von Epoxid-Ringöffnungsprodukten beispielsweise aus epoxidierten ungesättigten Fettsäureestern und Fettalkoholpolyglykolethern unter Verwendung von Phosphorsäure, phosphoriger Säure oder unterphosporiger Säure als Katalysatoren.

In der DE-A 42 33 219 (3) werden innenständige Hydroxymischether beschrieben, die durch Ringöffnung von Epoxiden von ungesättigten Fettsäureestern mit Fettalkoholpolyglykolethern in Gegenwart basischer Katalysatoren wie Natriummethylat hergestellt werden. Derartige innenständige Hydroxymischether werden als Hilfsmittel zur Entwässerung von feinteiligen Feststoffsuspensionen empfohlen.

Derartige Produkte wie die aus (1) bis (3) bekannten Verbindungen zeigen, wenn man sie als Entschäumer bei den verschiedensten technischen Anwendungen einsetzt, ebenfalls eine noch unbefriedigende Entschäumerwirkung. Aufgabe der vorliegenden Erfindung war es daher, Verbindungen bereitzustellen, die eine bessere Entschäumerwirkung aufweisen.

Demgemäß wurden Hydroxymischether gefunden, welche dadurch erhältlich sind, daß man Epoxide von ungesättigten Fettsäureestern einer ringöffnenden Umsetzung mit Polyglykolethern der allgemeinen Formel I

$$R^1—O—(CH_2—CHR^2—O)_n—H \qquad (I)$$

in der $R^1$ für Wasserstoff, $C_1$- bis $C_{30}$-Alkyl oder $C_8$- bis $C_{30}$-Alkenyl steht, $R^2$ Wasserstoff, Methyl oder Ethyl bezeichnet und n eine Zahl von 1 bis 50, vorzugsweise 2 bis 30, insbesondere 3 bis 20, vor allem 5 bis 10, für den Fall $R^1 \neq$ Wasserstoff oder eine Zahl von 2 bis 100, vorzugsweise 3 bis 70, insbesondere 5 bis 50, vor allem 10 bis 30, für den Fall $R^1$ = Wasserstoff bedeutet, in Gegenwart von Lewis-Säuren mit Ausnahme von Protonen unterwirft.

Lewis-Säuren sind Elektronenpaarakzeptoren und zeigen somit die Natur von Elektrophilen. Sie treten als Kationen, insbesondere Metallkationen, und koordinativ ungesättigte Verbindungen auf. Protonen ($H^{\oplus}$) werden im Sinne der vorliegenden Erfindung von dieser Definition ausgenommen.

Typische Beispiele für einsetzbare Lewis-Säuren sind Aluminiumtrichlorid, Aluminiumtribromid, Bortrichlorid, Phosphortrichlorid, Phosphortribromid, Antimontrichlorid, Arsentrichlorid, Eisentrichlorid, Eisentrifluorid, Zinkdichlorid, Zinndichlorid, Zinntetrachlorid, Titantetrachlorid, Silber(I)-nitrit sowie Aluminiumalkyle. Es können auch Gemische der genannten Lewis-Säuren verwendet werden.

In einer bevorzugten Ausführungsform werden als Lewis-Sauren Bortrifluorid, Bortrifluorid-Addukte oder Fluoroborsäure oder auch Gemische hieraus zur ringöffnenden Umsetzung der Epoxide mit den Polyglykolethern eingesetzt.

Dabei kann die Lewis-Säure Bortrifluorid ($BF_3$) als solche, z.B. unverdünnt gasförmig oder verdünnt mit Inertgasen wie Stickstoff oder inerten organische Lösungsmitteln wie Kohlenwasserstoffen, oder vorzugsweise in Form ihrer Addukte, insbesondere an Wasser, Alkohole, Ether, Ammoniak, Amine, Cyanverbindungen oder Phosphine, eingesetzt werden. Ein besonders bevorzugtes Addukt ist das Diethylether-Addukt $BF_3 . (C_2H_5)_2O$ ("Bortrifluoridetherat"). Auch Fluoroborsäure ($HBF_4$) kann eingesetzt werden, welche normalerweise als wäßrige Lösung verwendet wird. Aber auch das im wasserfreien Zustand auftretende Oxoniumsalz der Fluoroborsäure ($H_3O[BF_4]$) und Hydrolyseprodukte der wäßrigen Fluoroborsäure (z.B. $HBF_3(OH)$) sind erfindungsgemäß wirksam.

Die ringöffnende Umsetzung der Epoxide mit den Polyglykolethern wird in der Regel bei Temperaturen von 0 bis 75°C, insbesondere 15 bis 55°C, vor allem 20 bis 50°C durchgeführt; die benötigten Temperaturen sind damit deutlich niedriger als bei den aus dem Stand der Technik bekannten Herstellungsverfahren für derartige Systeme mit anderen Katalysatoren.

Der Lewis-Säure-Katalysator wird in der hierfür üblichen Menge eingesetzt, d.h. etwa von 0,1 bis 8 Gew.-%, insbesondere 0,5 bis 3 Gew.-%, bezogen auf das Epoxid.

Die Umsetzung der Ausgangsmaterialien erfolgt meistens unverdünnt, man kann aber auch in inerten organischen Lösungsmitteln arbeiten. In aller Regel arbeitet man unter Normaldruck.

Bei den erfindungsgemäßen Hydroxymischethern handelt es sich um neue Substanzen. Sie weisen eine typische Oligomerenverteilung, beispielsweise im Molekulargewichtsbereich von ca. 5000 bis ca. 8000 für die nachfolgend angeführten experimentellen Beispiele, sowie typische hohe Viskositätsbereiche auf. Verglichen mit den entsprechend basisch katalysiert hergestellten innenständigen Hydroxymischethern aus (3) weisen sie erfindungsgemäßen Produkte deutlich höhere Molekulargewichte und Viskositäten auf, was anwendungstechnisch von Vorteil ist.

Als Nucleophil für die Ringöffnung der Epoxide eignen sich die beschriebenen Polyglykolether I. Hierbei handelt es sich um Ethoxylate, Propoxylate oder Butoxylate von gesättigten oder ungesättigten Alkoholen oder um Ethylenoxid-, Propylenoxid- oder Butylenoxid-Blockpolymerisate. Natürlich können auch gemischte Alkoholethoxylate/-propoxylate oder Alkoholethoxylate/-butoxylate oder Alkoholpropoxylate/-butoxylate bzw. Ethylenoxid/Propylenoxid- oder Ethylenoxid/Butylenoxid- oder Propylenoxid/Butylenoxid-Blockcopolymerisate verwendet werden.

Der Rest $R^1$ in den Polyglykolethern I steht vorzugsweise für lineares oder verzweigtes $C_1$- bis $C_{22}$-Alkyl, insbesondere $C_4$- bis $C_8$-Alkyl, z.B. Methyl, Ethyl, n-Propyl, iso-Propyl, n-Butyl, iso-Butyl, sek.-Butyl, tert.-Butyl, n-Pentyl, n-Hexyl, n-Heptyl, n-Octyl, 2-Ethylhexyl, iso-Nonyl, n-Decyl, n-Dodecyl, n-Tetradecyl, iso-Tridecyl, Hexadecyl, Octadecyl oder Eicosyl.

Steht $R^1$ für $C_8$- bis $C_{30}$-Alkenyl, sind damit vornehmlich ungesättigte Fettalkoholreste wie Oleyl, Elaidyl, Petroselinyl, Linolyl, Linolenyl, Gadoleyl oder Erucyl gemeint. $R^1$ kann weiterhin auch für Mischungen gesättigter, ungesättigter oder gesättigter und ungesättigter Reste stehen, wie sie beispielsweise in der Natur vorkommen, z.B. Kokosfettalkoholschnitte, oder bei technischen Synthesen, z.B. der Oxo-Synthese, anfallen.

In einer bevorzugten Ausführungsform setzt man Polyglykolether der allgemeinen Formel Ia

$$R^8{-}O{-}(CH_2{-}CHR^2{-}O)_m{-}H \qquad \text{(Ia)}$$

in der $R^2$ Wasserstoff, Methyl oder Ethyl bezeichnet, $R^8$ für $C_4$- bis $C_8$-Alkyl, insbesondere für n-Butyl, steht und m eine Zahl von 5 bis 15, insbesondere 6 bis 10, bedeutet, ein.

Unter Epoxiden von ungesättigten Fettsäureestern sind in einer bevorzugten Ausführungsform Epoxide von ungesättigten Fettsäureniedrigalkylestern der allgemeinen Formel II

$$R^3CO{-}OR^4 \qquad \text{(II)}$$

in der $R^3CO$ für den Acylrest einer $C_8$- bis $C_{30}$-Alkenmonocarbonsäure und $R^4$ für einen $C_1$- bis $C_4$-Alkylrest wie Methyl, Ethyl, n-Propyl, iso-Propyl, n-Butyl, iso-Butyl, sek.-Butyl oder tert.-Butyl steht, zu verstehen.

Als Alkenmonocarbonsäuren mit 8 bis 30, insbesondere 12 bis 26, vor allem 16 bis 22 C-Atomen kommen beispielsweise die entsprechenden ein- oder mehrfach ungesättigen Fettsäuren in Betracht, z.B. Palmoleinsäure, Ölsäure, Elaidinsäure, Petroselinsäure, Linolsäure, Linolensäure, Gadoleinsäure, Erucasäure sowie deren technische Mischungen.

Die eingesetzten Epoxide der ungesättigten Fettsäureniedrigalkylester können vollständig epoxidiert vorliegen, sie können jedoch auch noch Doppelbindungen enthalten. Vorzugsweise liegt der Epoxidierungsgrad, bezogen auf die zur Verfügung stehenden Doppelbindungen, bei 50 bis 100 % und insbesondere bei 70 bis 95 %. Da zur Herstellung der epoxidierten Fettsäureester meist technische Schnitte oder Mischungen ungesättigter Fettsäureniedrigalkylester herangezogen werden, die noch gesättigte Anteile enthalten können, können die eingesetzten Epoxide folglich ebenfalls auch noch geringe Anteile gesättiger Fettsäureniedrigalkylester aufweisen. Ein besonders bevorzugtes Einsatzmaterial ist Ölsäureniedrigalkylesterepoxid, insbesondere Ölsäuremethylesterepoxid, mit einem Epoxidsauerstoffgehalt von 3 bis 6 Gew.-%, insbesondere 4,5 bis 5,5 Gew.-%.

Unter Epoxiden von ungesättigten Fettsäureestern sind in einer weiteren bevorzugten Ausführungsform Epoxide von ungesättigten Fettsäureglycerinestern der allgemeinen Formel III

$$\begin{array}{l} CH_2-O-COR^5 \\ | \\ CH-O-COR^6 \\ | \\ CH_2-O-COR^7 \end{array} \qquad (III)$$

in der $R^5CO$, $R^6CO$ und $R^7CO$ unabhängig voneinander im wesentlichen für Acylreste von $C_8$- bis $C_{30}$-Alkenmonocarbonsäuren stehen.

Als $C_8$- bis $C_{30}$-Alkenmonocarbonsäuren kommen die gleichen ungesättigten Säuren wie oben genannt in Betracht.

Typische Beispiele für derartige Epoxide sind Epoxide von ungesättigten Triglyceriden pflanzlicher oder tierischer Herkunft wie Sojaöl, Rüböl, Olivenöl, Sonnenblumenöl, Baumwollsaatöl, Erdnußöl, Leinöl, Rapsöl, Rindertalg oder Fischöl. Auch diese Einsatzstoffe können gesättigte Anteile enthalten. Meist werden Epoxide solcher Fettsäureglycerinester eingesetzt, die eine Jodzahl im Bereich von ca. 50 bis ca. 150, insbesondere 80 bis 120 aufweisen. Wie schon zuvor geschildert, kommen als Einsatzstoffe sowohl vollständig wie auch partiell epoxidierte Ester in Betracht. Ein besonders bevorzugtes Einsatzmaterial ist epoxidiertes Sojaöl mit einem Epoxidsauerstoffgehalt von 4 bis 7,5 Gew.-%, insbesondere 4,5 bis 6,5 Gew.-%.

Üblicherweise können die Epoxide der ungesättigten Fettsäureester und die Polyglykolether I bzw. Ia zur Herstellung der erfindungsgemäßen Hydroxymischether im molaren Verhältnis von 1:0,3 bis 1:1,5, insbesondere von 1:0,5 bis 1:1,1, bezogen auf den Epoxidgehalt, eingesetzt werden.

Gegenstand der vorliegenden Erfindung ist auch ein Verfahren zur Herstellung von Hydroxymischethern durch ringöffnende Umsetzung von Epoxiden von ungesättigten Fettsäureestern mit Polyglykolethern der allgemeinen Formel I

$$R^1-O-(CH_2-CHR^2-O)_n-H \qquad (I)$$

in der $R^1$ für Wasserstoff, $C_1$- bis $C_{30}$-Alkyl oder $C_8$- bis $c_{30}$-Alkenyl steht, $R^2$ Wasserstoff, Methyl oder Ethyl bezeichnet und n eine Zahl von 1 bis 50 für den Fall $R^1 \neq$ Wasserstoff oder eine Zahl von 2 bis 100, für den Fall $R^1$ = Wasserstoff bedeutet, welches dadurch gekennzeichnet ist, daß man die ringöffnende Umsetzung in Gegenwart von Lewis-Säuren mit Ausnahme von Protonen durchführt.

Die erfindungsgemäßen Hydroxymischether eignen sich in hervorragender Weise als biologisch abbaubare Entschäumer bei einer Vielzahl von technischen Anwendungen. Insbesondere können sie als Entschäumer in der Nahrungsmittelindustrie und bei Fermentationsprozessen, in Wasch- und Reinigungsmitteln, bei der Textilherstellung, bei der Leder- und Pelzherstellung, bei der Papierherstellung, bei der Metallbearbeitung und bei der Erdölgewinnung eingesetzt werden.

Als Einsatzgebiete in der Nahrungsmittelindustrie kommen insbesondere die Verarbeitung zuckerhaltiger Pflanzensäfte, wie sie in großem Maßstab bei der Zuckerrübenaufarbeitung erfolgt, die Kartoffelverarbeitung und die Herstellung von Kartoffelfertigprodukten wie Chips oder Pommes Frites sowie die alkoholische Gärung in Betracht. Als technische Fermentationsprozesse, bei denen die erfindungsgemäßen Hydroxymischether als Entschäumer eingesetzt werden können, sind beispielsweise die Erzeugung von Hefen wie Backhefe unter Verwendung von Melasse und die Gewinnung von Arzneimitteln durch Fermentation zu nennen.

Als Einsatzgebiete bei Wasch- und Reinigungsmitteln sind vor allem maschinelle Geschirrspülmittel, Textilwaschmittel sowie die industrielle maschinelle Flaschenreinigung zu nennen.

Bei der Textilherstellung ist der Einsatz von Entschäumern beim Wasch-, Bleich- und Färbevorgang notwendig. So können die erfindungsgemäßen Hydroxymischether hier insbesondere in Vorreinigungs- und Entschlichtungsbädern, aber auch beim Schichtungsschritt selber, in Mercerisierungslaugen, in Färbebädern sowie beim Textildruck in Druckpasten verwendet werden.

Bei der Papierherstellung sind Entschäumer ebenfalls ein wichtiges Hilfsmittel, aber auch beim Papierdruck analog zum Textildruck ist ihr Einsatz möglich. Aufgrund eines hohen Luftgehaltes im Wasserkreislauf von Papiermaschinen können infolge von Schaumbildung bei der Papierfabrikation mannigfache Störungen auftreten. Wenn dabei der Schaum mit flotiertem Schmutz während der Blattbildung auf die Papierbahn gelangt, entstehen auf dem Papier Schaumflecken. Der Schaum, der sich bei der Papierherstellung bildet, ist in der Regel stabil und hält sich daher lange an strömungsarmen Stellen im Papiermaschinenkreis auf, an denen er eintrocknen und in dieser Form vom Papierstoff weggetragen werden kann. Bei der Blattbildung entstehen dann ebenfalls Flecken im Papier. Man verwendet daher meist Entschäumer, die die Neigung haben, sich an der Phasengrenze Luft/Wasser anzureichern und somit einer

Schaumbildung entgegenwirken. Die Luftblasen stören beispielsweise den Entwässerungsvorgang des Papierstoffs auf der Papiermaschine, verursachen eine poröse Struktur des Blatts und können sogar Löcher in der Papierbahn bilden. Da bei der Papierherstellung aus Rationalisierungsgründen immer höhere Arbeitsgeschwindigkeiten auf den Maschinen angewendet werden, steigt naturgemäß die Gefahr der Untermischung von Luft in die Fasersuspension. Da weiterhin die Wasserkreisläufe der Papiermaschinen immer stärker geschlossen werden, reichern sich darin wasserlösliche Stoffe an, die den Schaum stabilisieren und teilweise sogar auch die Schaumbildung fördern. Außerdem erhöht sich dadurch die Temperatur des im Kreise geführten Wassers der Papiermaschine, auch diese Temperaturerhöhung hat einen Einfluß auf die Wirkung von Entschäumern. - Die erfindungsgemäßen Hydroxymischether werden allen diesen Anforderungen für Entschäumer bei der Papierherstellung im wesentlichen gerecht.

Da bei der Förderung und Aufarbeitung von Erdöl das Zweiphasengemisch Rohöl-Wasser, welches in der Regel oberflächenaktive Stoffe enthält, intensiv mechanisch beansprucht wird, muß auch hier entschäumt bzw. der Schaumbildung vorgebeugt werden, wozu sich die erfindungsgemäßen Hydroxymischether ebenfalls gut eignen.

Die erfindungsgemäßen Hydroxymischether zeigen durchweg eine hervorragende Entschäumerwirkung. Sie können sowohl vor oder nach dem Auftreten von Schaum zugegeben werden. Sie zeigen gute Spontan- und Langzeitwirkung über den gesamten pH-Bereich sowie bei höheren und niederen Temperaturen. Aufgrund der erfindungsgemäßen Herstellweise weisen sie eine typische Oligomerenverteilung mit hohem Oligomeranteil und damit hohe Vikositätswerte auf, was sich günstig auf die Entschäumerwirkung auswirkt.

Die erfindungsgemäßen Hydroxymischether sind biologisch gut abbaubar, sie liefern in der Regel im Standardversuch nach Zahn-Wellens DOC-Werte (nach 16 Tagen) von mindestens 70 bis 80 %.

Die erfindungsgemäßen Hydroxymischether sind in der Regel geruchlos, geschmacklos und physiologisch unbedenklich und können somit normalerweise problemlos bei der Herstellung von Lebensmitteln und Arzneimitteln eingesetzt werden. Außerdem können sie in den meisten Fällen ohne Probleme von dem zu entschäumenden Gut wieder abgetrennt werden.

## Beispiele

### Beispiel 1

Umsetzung von epoxidiertem Sojaöl mit n-Butanol + 9,1 mol Ethylenoxid

237 g des Alkohols (n-Butanol mit 9,1 mol Ethylenoxid) wurden in einem Reaktionsgefäß bei 25°C vorgelegt und unter Stickstoff mit 2 g Bortrifluoridetherat versetzt. Unter Rühren fügte man tropfenweise 122 g epoxidiertes Sojaöl (6,5 Gew.-% Epoxid) hinzu. Anschließend wurde die Reaktionstemperatur auf 50°C erhöht und man rührte drei Stunden nach. - Als Produkt erhielt man ein hellgelbes Öl (350 g) mit einer Viskosität (nach Ubbelohde) von 630 mPa • s.

### Beispiel 2

Umsetzung von epoxidiertem Ölsäuremethylester mit n-Butanol + 6 mol Ethylenoxid

Die Reaktion erfolgte analog Beispiel 1 unter Einsatz von 26,1 g n-Butanol + 6 mol Ethylenoxid, o,5 g Bortrifluoridetherat und 25,3 g epoxidiertem Ölsäuremethylester (5,1 Gew.-% Epoxid). - Ausbeute: 50 g eines hellgelben Öls.

### Beispiel 3

"Zuckertest" für die Verbindungen der Beispiele 1 und 2

Die anwendungstechnischen Daten für die Entschäumerwirkung wurden im "Zuckertest" ermittelt, dessen Ausführung im folgenden beschrieben ist:

Die Zuckertestapparatur besteht aus einem graduierten Standzylinder, dessen Boden aus einer Fritte (Porengröße: D2) besteht. In den Zylinder werden 110 ml einer Lösung aus 150 g Zucker, 0,33 g Saponin (pflanzliches Glykosid als Schaumbildner) und 1050 ml Wasser gefüllt. Anschließend wird durch die Fritte eine Luftstrom von 660 ml/min geleitet. Man läßt den Schaum auf die Gesamthöhe von 500 ml ansteigen und gibt dann mit Hilfe einer Pipette 0,02 ml Entschäumer direkt in die Lösung. Um die Spontanwirkung zu messen, wird die Zeit festgestellt, die der Entschäumer zur Schaumzerstörung benötigt. Die Langzeitwirkung des Entschäumers wird durch Ablesen der Schaumhöhe nach 1, 5 und 10 Minuten ermittelt.

Ergebnisse:

| Substanz | Schaumhöhe [ml] | | |
|---|---|---|---|
| | nach 1 Minute* | nach 5 Minuten* | nach 10 Minuten* |
| aus Beispiel 1 | 12 | 12 | 46 |
| aus Beispiel 2 | 20 | 38 | 42 |
| zum Vergleich: handelsübliches Ethylenoxid/Propylenoxid-Blockpolymer (Gew.-Verh. 10:90, Molmasse 1950) | 270 | 475 | >500 |
| zum Vergleich: handelsübliches Ethylenoxid/Propylenoxid-Blockpolymer (Gew.-Verh. 10:90, Molmasse 2250) | 200 | 200 | 200 |

* jeweils nach Zugabe des Entschäumers

Vergleichsbeispiel A

Beispiel 1 wurde wiederholt mit dem Unterschied, daß die Umsetzung gemäß (3) mit 0,5 g Natriummethylat katalysiert wurde. - Als Produkt erhielt man ein gelbliches Öl (130 g) mit einer Viskosität von 182 mPa • s (nach Ubbelohde). Die im Zuckertest ermittelten Schaumhöhen betrugen 330 ml (1 Min.), 190 ml (5 Min.), 170 ml (10 Min.).

Beispiel 4

Umsetzung von epoxidiertem Sojaöl mit n-Butanol + 6,4 mol Ethylenoxid

85,8 g des Alkohols wurden in einem Reaktionsgefäß bei 25°C vorgelegt und unter Stickstoff mit 1,5 g Bortrifluoridetherat versetzt. Unter Rühren fügte man tropfenweise 75,6 g epoxidiertes Sojaöl (6,5 Gew.-% Epoxid) hinzu. Anschließend wurde die Reaktionstemperatur auf 50°C erhöht und man rührte acht Stunden nach. - Als Produkt erhielt man ein hellgelbes Öl (152 g), Schaumhöhen: 20 ml (1 Min.), 20 ml (5 Min.), 30 ml (10 Min.), Viskosität: 1562 mPa • s (nach Ubbelohde).

Beispiel 5

Umsetzung von epoxidiertem Sojaöl mit n-Butanol + 9,1 mol Ethylenoxid

71,1 g des Alkohols wurden in einem Reaktionsgefäß bei 25°C vorgelegt und unter Stickstoff mit 0,8 g Bortrifluoridetherat versetzt. Unter Rühren fügte man tropfenweise 75,8 g epoxidiertes Sojaöl (6,5 Gew.-% Epoxid) hinzu. Man rührte vier Stunden bei 25°C nach. - Als Produkt erhielt man ein hellgelbes Öl (131 g), Schaumhöhen: 15 ml (1 Min.), 20 ml (5 Min.), 25 ml (10 Min.), Viskosität: 3920 mPa • s (nach Ubbelohde).

Beispiel 6

Umsetzung von epoxidiertem Sojaöl mit n-Butanol + 10 mol Ethylenoxid - Bestimmung der Schaumdämpfung in der Geschirrspülmaschine

157,3 g des Alkohols wurden in einem Reaktionsgefäß bei 25°C vorgelegt und unter Stickstoff mit 1 g Bortrifluoridetherat versetzt. Unter Rühren fügte man tropfenweise 75,6 g epoxidiertes Sojaöl (6,5 Gew.-% Epoxid) hinzu. Man rührte anschließend 3,5 Stunden bei 25°C nach und erhielt ein hellgelbes Öl. - Die Prüfung des Produktes in der Geschirrspülmaschine ("Ei-Test") ergab eine ausgezeichnete Schaumdämpfung (90 U/min). Bei Einsatz des Produktes aus Vergleichsbeispiel A resultierte eine deutlich schlechtere Schaumdämpfung (58 U/min).

Beim sogenannten "Ei-Test" zur Prüfung des Schaumdämpfungsverhaltens wird durch magnetische Induktionsmessung in einem handelsüblichen Haushalts-Geschirrspülautomaten mit Hilfe eines Zählwerks die Zahl der Umdrehungen eines Sprüharms bestimmt. Durch Schaumbildung, die besonders bei Anwesenheit von Proteinen (Eiweiß) auftritt, wird die Umdrehungszahl des Sprüharms vermindert. Die Umdrehungszahl stellt somit wegen der verringerten Rückstoßkraft ein Maß für die Tauglichkeit von Tensiden in Reinigungsgeräten mit hoher Mechanik dar. Die Testzeit

beträgt 12 Minuten, wobei die durchschnittliche Umdrehungszahl pro Minute aus der Gesamtumdrehungszahl berechnet wird. Der Waschvorgang wird bei Raumtemperatur begonnen, nach etwa 10 Minuten beträgt die Temperatur des Spülwassers 60°C.

Beispiel 7

Bestimmung des Schaumwertes bei der Papierherstellung

In einer Rinne aus einem durchsichtigen Kunststoff wurden jeweils 5 l einer schaumentwickelnden Papierstoffsuspension (0,1 Gew.-% Holzschliff) 5 Minuten umgepumpt. Die an der Oberfläche der Stoffsuspension gebildete Schaummenge wurde dann mit Hilfe eines Rasters an der Wand der Rinne in Flächeneinheiten ($cm^2$) gemessen und als sogenannter Schaumwert zur Beurteilung der Wirksamkeit eines Entschäumers angegeben.

Pumpte man die Papierstoffsuspension in Abwesenheit eines Entschäumers um, so betrug der Schaumwert nach 5 Minuten 1250 $cm^2$. Durch die Zugabe von jeweils 2 mg/l eines wirksamen Entschäumers (insgesamt 10 mg) zu der Holzschliffsuspension wurde dieser Schaumwert deutlich reduziert, so daß er ein Maß für die Wirksamkeit eines Entschäumers darstellte.

Prüfung der Entschäumer

Die Temperatur der Papierstoffsuspension wurde mittels eines mit einem Regler verbundenen Tauchsieders konstant gehalten und betrug 50°C. Die Temperatur der Stoffsuspension blieb während der Prüfung konstant auf 50 ± 1°C.

Die Wirksamkeit der Entschäumer wird als % Restschaum dargestellt.

Der % Restschaum (R) wird berechnet als

$$R = \frac{S_e \cdot 100}{S_o}$$

wobei $S_e$ den Schaumwert bedeutet, der nach Zugabe des Entschäumers resultiert, und $S_o$ der Schaumnullwert ist, d.h. der Wert, der in Abwesenheit eines Entschäumers gemessen wird. In dieser Terminologie ist der Entschäumer um so besser, je kleiner der Wert von R ist.

Als Entschäumer wurden das Produkt aus Vergleichsbeispiel A und das Produkt aus Beispiel 5 eingesetzt. Das Produkt aus Vergleichsbeispiel A ergab R = 51 % Restschaum, das Produkt aus Beispiel 5 ergab R = 42 % Restschaum.

## Patentansprüche

1. Hydroxymischether, dadurch erhältlich, daß man Epoxide von ungesättigten Fettsäureestern einer ringöffnenden Umsetzung mit Polyglykolethern der allgemeinen Formel I

$$R^1\text{—O—}(CH_2\text{—}CHR^2\text{—}O)_n\text{—}H \qquad (I)$$

in der $R^1$ für Wasserstoff, $C_1$- bis $C_{30}$-Alkyl oder $C_8$- bis $C_{30}$-Alkenyl steht, $R^2$ Wasserstoff, Methyl oder Ethyl bezeichnet und n eine Zahl von 1 bis 50 für den Fall $R^1 \neq$ Wasserstoff oder eine Zahl von 2 bis 100 für den Fall $R^1$ = Wasserstoff bedeutet, in Gegenwart von Lewis-Säuren mit Ausnahme von Protonen unterwirft.

2. Verfahren zur Herstellung von Hydroxymischethern durch ringöffnende Umsetzung von Epoxiden von ungesättigten Fettsäureestern mit Polyglykolethern der allgemeinen Formel I

$$R^1\text{—O—}(CH_2\text{—}CHR^2\text{—}O)_n\text{—}H \qquad (I)$$

in der $R^1$ für Wasserstoff, $C_1$- bis $C_{30}$-Alkyl oder $C_8$- bis $C_{30}$-Alkenyl steht, $R^2$ Wasserstoff, Methyl oder Ethyl bezeichnet und n eine Zahl von 1 bis 50 für den Fall $R^1 \neq$ Wasserstoff oder eine Zahl von 2 bis 100 für den Fall $R^1$ = Wasserstoff bedeutet, dadurch gekennzeichnet, daß man die ringöffnende Umsetzung in Gegenwart von Lewis-Säuren mit Ausnahme von Protonen durchführt.

3. Verfahren zur Herstellung von Hydroxymischethern nach Anspruch 2, dadurch gekennzeichnet, daß man die ringöffnende Umsetzung in Gegenwart von Bortrifluorid, Bortrifluorid-Addukten oder Fluoroborsäure durchführt.

4. Verfahren zur Herstellung von Hydroxymischethern nach Anspruch 2 oder 3 durch ringöffnende Umsetzung von

Epoxiden von ungesättigten Fettsäureniedrigalkylestern der allgemeinen Formel II

$$R^3CO—OR^4 \qquad (II)$$

in der $R^3CO$ für den Acylrest einer $C_8$- bis $C_{30}$-Alkenmonocarbonsäure und $R^4$ für einen $C_1$- bis $C_4$-Alkylrest steht.

**5.** Verfahren zur Herstellung von Hydroxymischethern nach Anspruch 2 oder 3 durch ringöffnende Umsetzung von Epoxiden von ungesättigten Fettsäureglycerinestern der allgemeinen Formel III

$$\begin{array}{l} CH_2—O—COR^5 \\ | \\ CH—O—COR^6 \\ | \\ CH_2—O—COR^7 \end{array} \qquad (III)$$

in der $R^5CO$, $R^6CO$ und $R^7CO$ unabhängig voneinander im wesentlichen für Acylreste von $C_8$- bis $C_{30}$-Alkenmonocarbonsäuren stehen.

**6.** Verfahren zur Herstellung von Hydroxymischethern nach den Ansprüchen 2 bis 5 durch ringöffnende Umsetzung mit Polyglykolethern der allgemeinen Formel Ia

$$R^8—O—(CH_2—CHR^2—O)_m—H \qquad (Ia)$$

in der $R^2$ Wasserstoff, Methyl oder Ethyl bezeichnet, $R^8$ für $C_4$- bis $C_8$-Alkyl steht und m eine Zahl von 5 bis 15 bedeutet.

**7.** Verfahren zur Herstellung von Hydroxymischethern nach den Ansprüchen 2 bis 6, dadurch gekennzeichnet, daß man die ringöffnende Umsetzung bei Temperaturen von 0 bis 75°C durchführt.

**8.** Verwendung von Hydroxymischethern gemäß Anspruch 1 oder von gemäß den Ansprüchen 2 bis 7 hergestellten Hydroxymischethern als biologisch abbaubare Entschäumer.

**9.** Verwendung von Hydroxymischethern nach Anspruch 8 als Entschäumer in der Nahrungsmittelindustrie und bei Fermentationsprozessen, in Wasch- und Reinigungsmitteln, bei der Textilherstellung, bei der Leder- und Pelzherstellung, bei der Papierherstellung, bei der Metallbearbeitung und bei der Erdölgewinnung.